# EUROPEAN PATENT APPLICATION

(11) **EP 4 209 194 A1**
(43) Date of publication of application: **12.07.2023**
(21) Application number: 22217167.0
(22) Date of filing: 29.12.2022
(51) Int. Cl.: A61B 34/10

(54) **DENTAL IMPLANTATION ASSESSMENT METHOD FOR IMPLANT DENTISTRY**

(30) Priority: 05.01.2022 TW 111100482
(71) Applicant: Star Generation Limited, New Taipei City 244008 (TW)
(72) Inventor: CHEN, CHUN-LEON, 110202 New Taipei City (TW); CHEN, NINA, Las Vegas, NV, 89146 (US); CHEN, AUDREE, Las Vegas, NV, 89146 (US); CHEN, NICHOLAS, 89146 Las Vegas, NV (TW); CHEN, ALEC, 89146 Las Vegas, NV (TW); CHEN, NOLAN, 89146 Las Vegas, NV (TW)
(74) Representative: Cabinet Chaillot

(57) **Abstract**

A dental implantation assessment method includes the steps of creating a 3D teeth model; setting up a reference line, creating a health status table, and forming a multi-level frame model for an implant placement position on the teeth model; dividing each level of the frame model into a centered implant module and multiple surrounding test modules; dividing each test module into multiple assessment zones, and combining the latter with the health status table; selecting and placing a dental implant model in the frame model, and defining one assessment zone in each test module that contacts with a periphery of the dental implant model as a selected zone; and lastly, computing assessment values assigned to the selected zones to determine an object type of the dental implant model. A dentist can decide an optimal dental implant through dental implant model size selection and implantation simulation to ensure successful dental implant surgery.

## Description

### FIELD OF THE INVENTION

The present invention relates to a dental implantation assessment method for checking whether a patient is allowed for immediate implant placement, and more particularly, to a dental implantation assessment method, in which a model is created in advance to simulate the connecting state between a dental implant and an alveolar bone area.

### BACKGROUND OF THE INVENTION

A person having one or more missing teeth owing to tooth decay, lesion or injury would often have problems in speaking, eating and good facial appearance. In this case, the manufacture of dentures for the position of a missing tooth is necessary. Currently, dental implants have gradually replaced the conventional dentures. In a dental implant surgery, a titanium artificial tooth root is implanted into a patient's alveolar bone to replace the damaged or missing tooth. It usually requires 3 to 6 months to get good osseointegration of dental implant. Thereafter, an artificial crown can be mounted on the dental implant to restore good facial appearance and chewing function.

While the dental implantation has become the main way of treating missing teeth, there are still risks of a failed dental implant surgery. There are many factors leading to a failed dental implant surgery, such as post-operative infection, bad occlusion habit, poor oral hygiene, poor implant design, etc. The problem of poor implant design is mainly attributable to the insufficient dentist experience that leads to incorrect selection of implant size, implant placement angle, implant placement direction, or implant placement position. Other problems attributable to inexperience dentist include wrong material for guided bone regeneration and incomplete wound treatment.

Among others, pre-surgery planning and good tooth treatment aiding appliances are important to reduce the risk of a failed dental implantation. In the conventional pre-surgery planning, radiography using X-ray is mainly used to get the patient's full-mouth X-ray film, based on which the dentist plans a general position for the dental implant and decides the desired implant placement direction and dental surgical guides on the patient's teeth model.

Following the constantly improved technologies, computed tomography (CT) scanning and simulating software can be now used to assist the dental in obtaining a three-dimensional (3D) teeth model that allows the dentist to observe from all directions. However, while the observation of the 3D teeth model image can help the dentist make his pre-surgical plan, there is not any software or application program presently available for assisting the dentist in judging whether a location of a missing tooth is allowed for implantation or which type of implant should be used at the location of a missing tooth to obtain the optimal dental implantation.

### SUMMARY OF THE INVENTION

A primary object of the present invention is to provide an assessment method for assisting a dentist in determining whether a dental implant can be used in an immediate implant placement and be firmly located in a patient's alveolar bone. According to the assessment method, the dentist first gets a patient's teeth model through computed tomography (CT) scan of the patient's oral areas and simulates the connecting state between a dental implant model and the patient's alveolar bone, so as to verify an object type of the dental implant model.

Another object of the present invention is to provide the above assessment method, in which, when more than one dental implant model is selected by the dentist, the selected dental implant model are analyzed based on the object types thereof to generate a suggested option to the dentist, enabling the dentist to easily determine a physical dental implant suitable for use in an immediate implant placement surgery.

A further object of the present invention is to provide the above assessment method, in which the object type of the dental implant model can be assessed according to a total number of zones having a valid value or a total number of zones having a required forcing strength. The dental implant model and its assessed object type are shown at the same time.

To achieve the above and other objects, the present invention provides a dental implantation assessment method for implant dentistry, which includes a model creation step S1, a position selection step S2, a table creation step S3, a frame forming step S4, a table-frame combination step S5, an implantation simulation step S6, and a result generation step S7.

In the model creation step, a teeth model having a three-dimensional (3D) appearance is created by scanning a patient's oral using X-ray computed tomography (CT). In the position selection step, an implant placement position is determined on the teeth model and a reference line passing a center of dental occlusion is set up at the implant placement position. In the table creation step, an alveolar bone area on the teeth model located around the reference line is determined on the electronic device and a health status table is generated by electronic device based on the alveolar bone area. In the frame forming step, a frame model is formed at the implant placement position around the reference line, such that the alveolar bone area is encircled by the frame model. The frame model internally defines a plurality of levels, each of the levels including an implant module in the center and a plurality of test modules located immediately around the implant module.

In the table-frame combination step, every test module is divided into a plurality of assessment zones, which are combined with the hardness data and the density data of health status table; an assessment value of the assessment zones are calculated by the electronic device according to the health status table. In the implantation simulation step, a dental implant model is selected for placement into the frame model, such that the implant module of the levels overlap the dental implant model and one of the assessment zones of every test module adjacent to an outer peripheral surface of the dental implant model is defined as a selected zone. In the result generation step, a computation is made on every selected zone to derive a sum value of the assessment values for determining an object type of the dental implant model; and the object type can be one of a suitable object type allowed for use in immediate implant placement and an unsuitable object type failing to provide stable and firm fixing ability.

In a preferred embodiment, the assessment method also includes a further simulation and assessment step and an option suggesting step. In the further simulation and assessment step, a replacement dental implant model is used in place of the dental implant model. The replacement dental implant model is different from the dental implant model in size and has a replacement outer peripheral surface. The assessment zones on every module adjacent to the replacement outer peripheral surface are defined as replacement selected zones, according to which an object type of the replacement dental implant model is determined.

In the option suggesting step, a comparison is made between the object type of the dental implant model and the replacement dental implant model to generate a suggested option. In the case the dental implant model and the replacement dental implant model all are determined as a suitable object, one of the suitable objects having a higher sum value of the assessment values is selected as the suggested option for use in the dental implant surgery; and in the case one of the dental implant model and the replacement dental implant model is determined as a suitable object while the other one is an unsuitable object, the one being the suitable object is selected as the suggested option for use in the dental implant surgery.

In the case both the dental implant model and the replacement dental implant model are determined as unsuitable objects, the suggested option includes an assessment result that no dental implant surgery can be performed and a re-simulation option that a re-simulation and assessment step is to be performed. In the re-simulation and assessment step of the assessment method, a further replacement dental implant model is used in place of the replacement dental implant model. The further replacement dental implant model is different from the replacement dental implant model in size and has a further replacement outer peripheral surface; one of the assessment zones on every test module adjacent to the further replacement outer peripheral surface are defined as a further replacement selected zone, so that an object type of the further replacement dental implant model is determined based on the further replacement selected zone of every test module.

In an operable embodiment, the frame model has a cross-sectional diameter that is limited by one of the following two measures: a distance between two teeth located immediately at two lateral sides of the implant placement position, and a width of gum of the teeth model. And, the dental implant model has an implant diameter and an implant length; the implant diameter limited by an area size of the frame model, and the implant length is limited by the number of levels of the frame model.

In the frame forming step, a height location of the frame model is determined according to a top 3D configuration of the alveolar bone area. The top 3D configuration defines a slanted reference surface, the slanted reference surface angularly intersects the reference line and the frame model has a top surface that passes an intersection of the slanted reference line and the reference line, such that the alveolar bone area is encircled by more than one half of the test modules in the uppermost level of the frame module.

Further, in the frame forming step, a tilting direction of the frame model is determined according to the health status of the alveolar bone area; and the frame model has a centerline that is inclined relative to the reference line, such that a plurality of contact volumes of the frame model with the alveolar bone area are changed to different positions.

The health status table has a plurality of hardness data and a plurality of density data for listing in different regions of the alveolar bone area, such that each of the assessment values in the corresponding assessment zone is decided to one of a valid value representing a healthy state and an invalid value representing an unhealthy state.

In another operable embodiment, the health status table has a plurality of hardness data and a plurality of density data for listing in different regions of the alveolar bone area, such that each of the assessment values is generated according to the hardness data and the density data and represents a forcing strength acting between the dental implant model and the alveolar bone area.

The assessment method of the present invention is characterized in that a CT scan is performed to obtain the health state of the patient's alveolar bone, so that the hardness and density at different locations of the teeth model can be organized into the health status table, based on which the connecting state between the dental implant model and teeth model can be simulated. Therefore, the dentist can select in the pre-surgery planning the optimal dental implant for use and reduce the chance of failure in a dental implant surgery.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein
Fig. 1 is a flowchart showing the steps included in the dental implantation assessment method according to a first preferred embodiment of the present invention;
Figs. 2A and 2B pictorially show a model creation step in the method of Fig. 1;
Fig. 3 pictorially shows a position selection step in the method of Fig. 1;
Fig. 4 pictorially shows a table creation step in the method of Fig. 1;
Fig. 5 pictorially shows a frame forming step in the method of Fig. 1;
Fig. 6 is a schematic view of a frame model formed in the present invention;
Fig. 7 pictorially shows the forming of a frame model based on a slanted reference surface of an alveolar bone area;
Fig. 8 shows a frame model being inclined relative to a reference line of an implant placement position;
Fig. 9 pictorially shows a table-frame combination step in the method of Fig. 1;
Figs. 10A and 10B pictorially show an implantation simulation step in the method of Fig. 1;
Figs. 11A to 11C pictorially show a result generation step in the method of Fig. 1;
Figs. 12A and 12B are mimic diagrams of different alveolar bone conditions;
Fig. 13 is a flowchart showing the steps included in the dental implantation assessment method according to a second preferred embodiment of the present invention;
Figs. 14A to 14C pictorially show a further simulation and assessment step in the method of Fig. 13;
Figs. 15A to 15C pictorially show an option suggesting step in the method of Fig. 13; and
Fig. 16 pictorially shows a re-simulation and assessment step in the method of Fig. 13.

### DETAILED DESCRIPTION OF THE PREFERRED EMBODIMENTS

The present invention will now be described with some preferred embodiments thereof and by referring to the accompanying drawings. For the purpose of easy to understand, elements that are the same in the preferred embodiments are denoted by the same reference numerals.

The present invention provides a dental implantation assessment method for implant dentistry, such that a dentist in planning a dental implant surgery can use an assistance program installed on an electronic device to assist in assessing whether an implant can be placed immediately after a tooth extraction and in deciding an optimal implant size to ensure a high successful rate of the dental implant surgery.

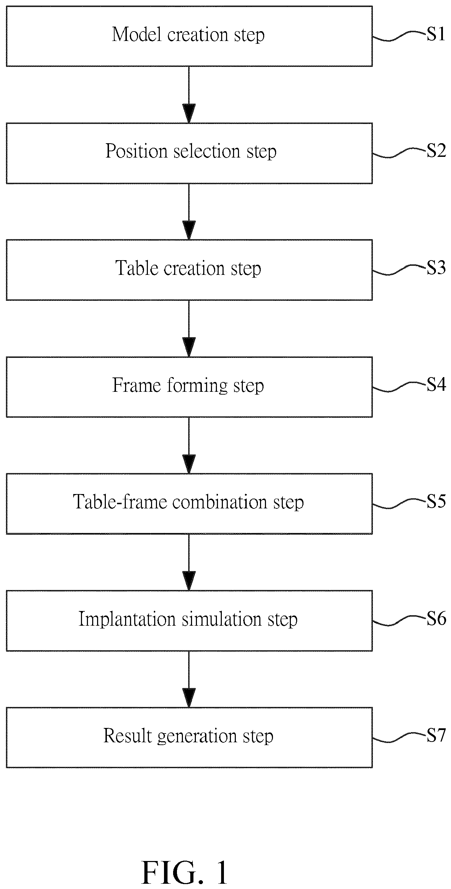

Please refer to Fig. 1. In a first preferred embodiment of the present invention, the dental implantation assessment method includes a model creation step S1, a position selection step S2, a table creation step S3, a frame forming step S4, a table-frame combination step S5, an implantation simulation step S6, and a result generation step S7.

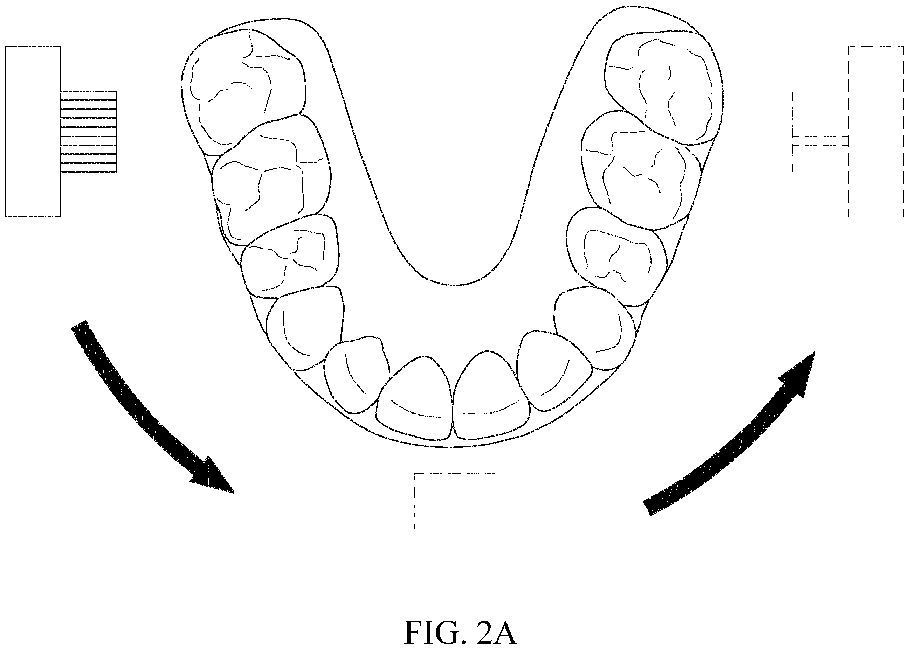

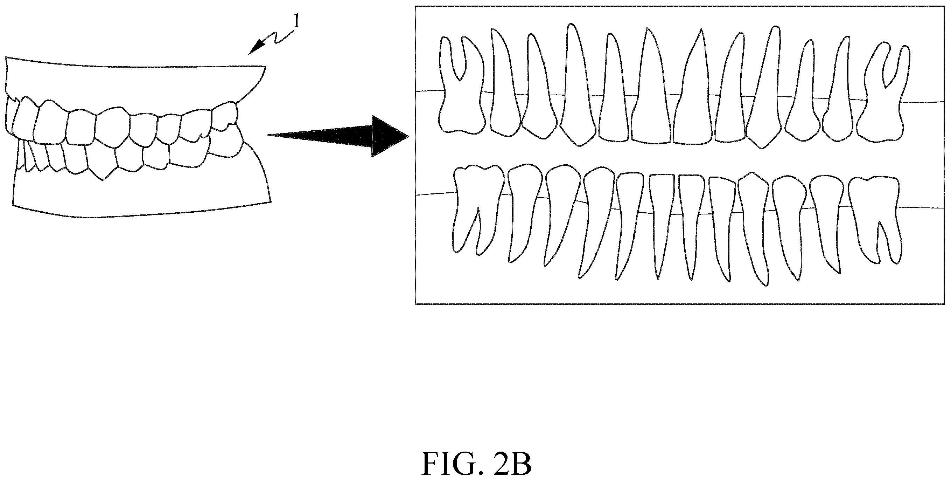

Please refer to Figs. 2A and 2B. In the module creation step S1, a computed tomography (CT) device is used to get a plurality of teeth images of a patient's oral areas. The electronic device further creates a teeth model 1 from the teeth images. The teeth model 1 can also be transformed into a planar image showing all teeth, as shown in Fig. 2B. In view that bone density of the patient's alveolar bone is an important factor having an influence on successful implant placement, the teeth model 1 shall present different colors according to different bone densities.

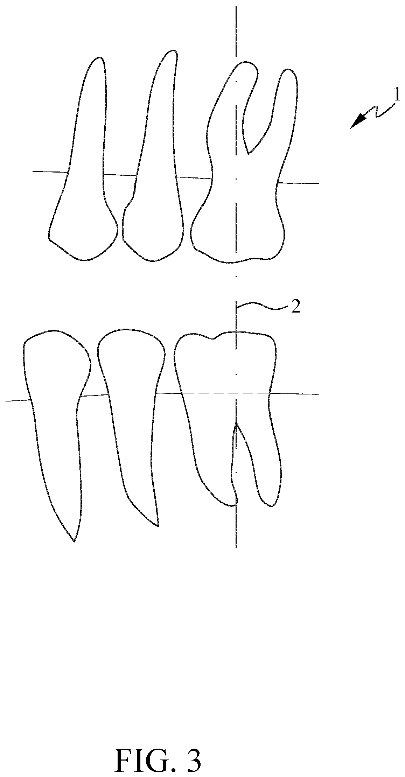

Please refer to Fig. 3. In the position determination step S2, the position of a damaged tooth is determined, so that an implant placement position on the teeth model can be selected by the dentist on the electronic device. Then, the electronic device shall set up a virtue reference line 2 that passes a center of the dental occlusion at the implant placement position.

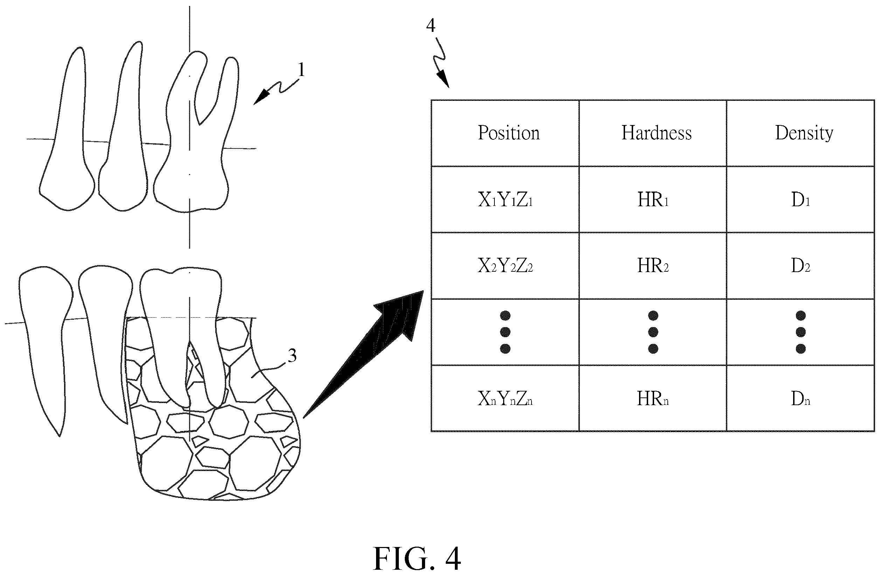

Please refer to Fig. 4. In the table creation step S3, an alveolar bone area 3 surrounding the root of the damaged tooth is determined based on the reference line 2 and the electronic device generates a health status table 4 according to the bone density of the alveolar bone area 3. As shown, the health status table 4 includes a position field, a hardness field, and a density field. In the position field, x-axis, y-axis and z-axis coordinates are used to define each of a plurality of region positions. The electronic device shall compute based on a color corresponding to the region position to obtain a hardness data and a density data, such that a plurality of position data, a plurality of hardness data, and a plurality of density data are listed in the health status table 4.

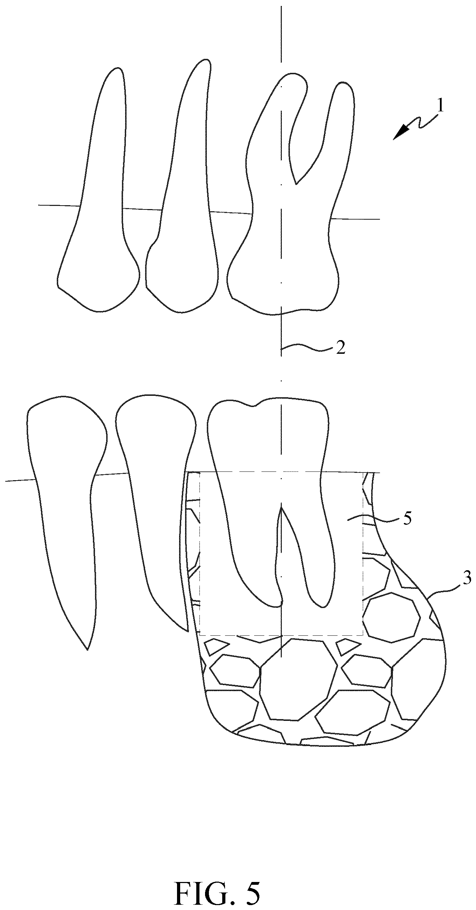

Please refer to Fig. 5. In the frame forming step S4, a frame model 5 is formed. The frame model 5 has a center in alignment with the reference line 2, such that the root of the damaged tooth and the alveolar bone area 3 are encircled by the frame model 5. A height location of the frame model 5 is determined according to a top 3D configuration of the alveolar bone area 3, and the frame model 5 has a cross-sectional diameter that is limited by one of the following two measures: a distance between two teeth located immediately at two lateral sides of the implant placement position, and a width of gum of the teeth model 1.

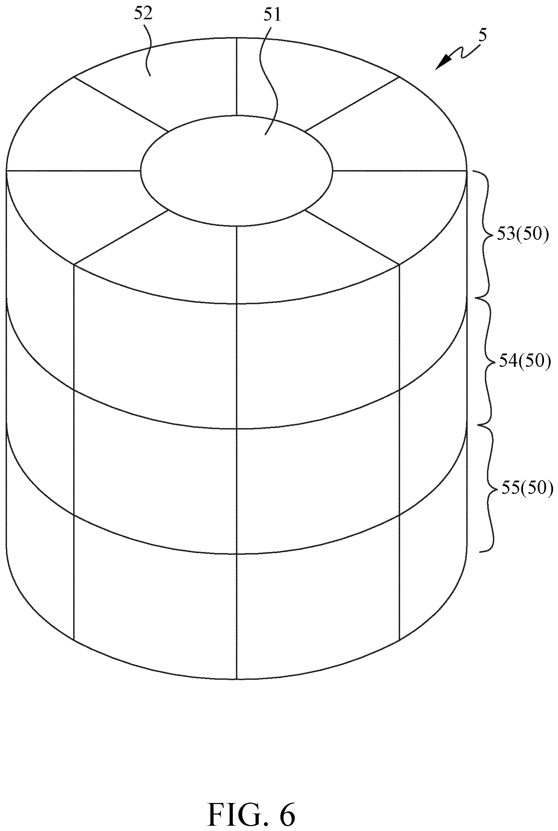

When the alveolar bone area 3 has an equal-height contour as shown in Fig. 5, the frame model 5 has a top flush with a reference surface of the alveolar bone area 3 and is then defined by the reference surface and the cross-sectional diameter to have a cylindrical shape, as shown in Fig. 6.

Please refer to Fig. 6. The frame model 5 internally defines a plurality of levels 50. Every level 50 includes an implant module 51 in the center and a plurality of test modules 52 located immediately around the implant module 51. The frame model 5 shown in Fig. 6 defines three levels 50, including an upper level 53, a middle level 54, and a lower level 55. The implant modules 51 of the three levels 50 are respectively defined as a round zone, and each of the round zones has eight sector-shaped test modules 52 located immediately around it.

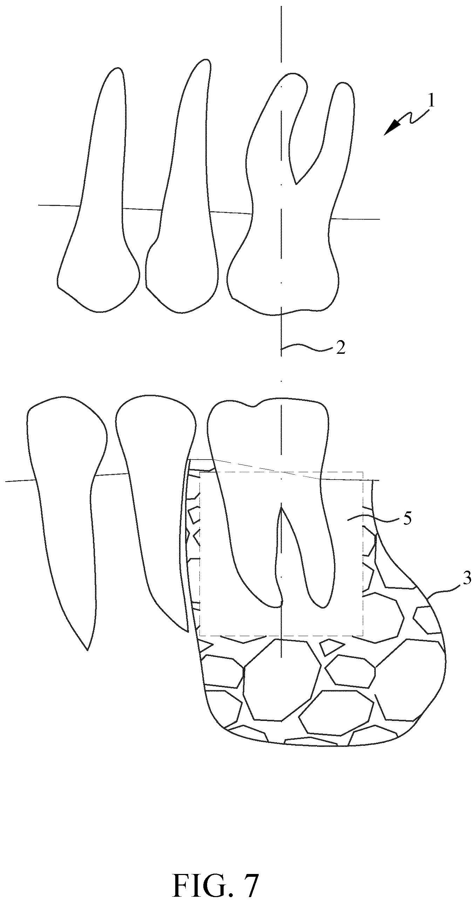

Please refer to Fig. 7. In another embodiment of the present invention, when the alveolar bone area 3 has a 3D configuration that is partially depressed, a slanted reference surface of the alveolar bone area 3 is defined based on the partially depressed 3D configuration, such that the slanted reference surface angularly intersects the reference line 2 and the frame model 5 shall have a top surface that passes an intersection of the slanted reference surface and the reference line 2. The frame model 5 is then defined by the top surface and the cross-sectional diameter, such that the alveolar bone area 3 is encircled by more than one half of the test modules 52 in the uppermost level 50.

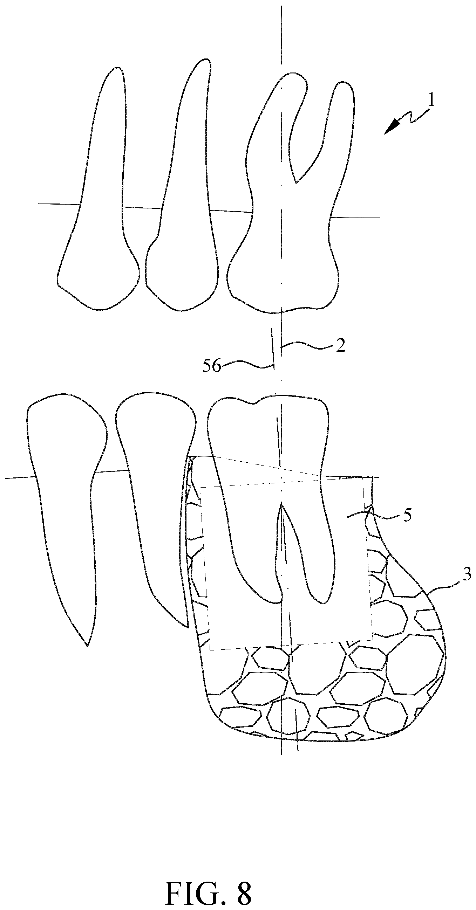

Please refer to Fig. 8. In the frame forming step S4, a tilting direction of the frame model 5 is determined according to the health status of the alveolar bone area 3. That is, the frame model 5 may have a centerline 56 that is inclined relative to the reference line 2, such that the frame model 5 is slanted relative to the reference line 2 and has a top center located on the reference line 2. With this arrangement, it is able to change proportions of contact of the frame model 5 with the alveolar bone area 3 in different directions, such that the frame model 5 can contact with more volume of the alveolar bone area 3 that has relatively good bone density and contact with less volume of the alveolar bone area 3 that has relatively poor bone density. By changing the frame model 5 to different angular positions, it allows the dentist to simulate and consider more possible patterns to decide the optimal way of dental implantation and increase the chance of performing the dental implant surgery successfully.

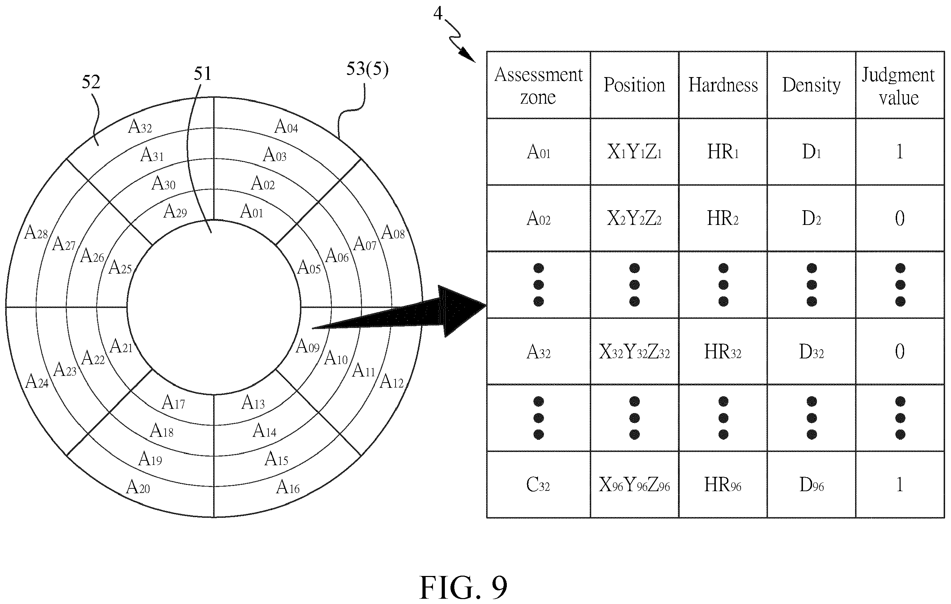

Please refer to Fig. 9. In the table-frame combination step S5, every test module 52 is divided into a plurality of assessment zones according to a radial distance between different zones of the test module 52 and the reference line 2. The assessment zones are assigned to different fields in the health status table 4, such that an assessment value is generated to each assessment zone according to the hardness data and the density data corresponding to the assessment zone. Wherein, the assessment zones in the upper level 53, the middle level 54 and the lower level 55 are numbered and given a leading letter "A", "B", and "C", respectively.

In the illustrated preferred embodiment, each of the test modules 52 in the three different levels 50 is divided into four assessment zones, which are sequentially located at different radial distances from the implant module 51. Therefore, all the assessment zones respectively have a specific spatial position and a set of corresponding position data, hardness data and density data. The electronic device shall compute the hardness data and the density data of each assessment zone to derive an assessment value for the same. As shown, the eight test modules 52 in the upper level 53 are divided into total 32 assessment zones, which respectively have an assessment value of [1] or [0]. Wherein, value [1] is a valid value indicating the bone in the corresponding assessment zone is in a healthy state, and value [0] is an invalid value indicating the bone in the corresponding assessment zone is in an unhealthy state.

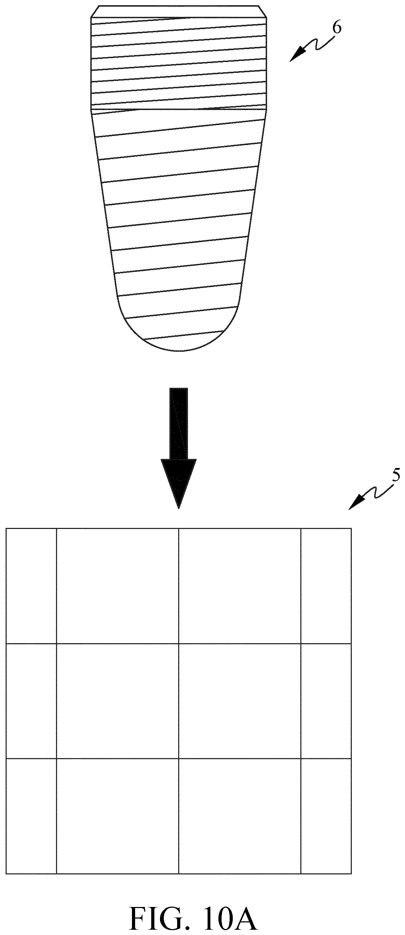

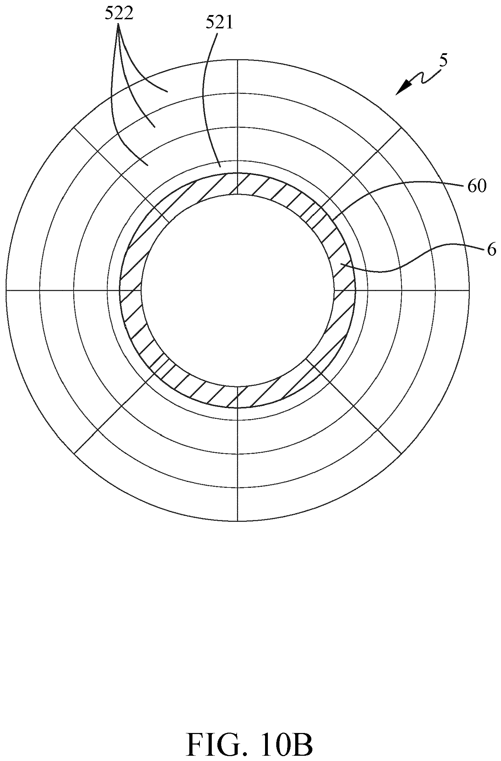

Please refer to Fig. 10A. In the implantation simulation step S6, the electronic device selects a dental implant model 6 for placing into the frame model 5. The dental implant model 6 is dimensionally larger than the implant modules 51 to therefore overlap all the implant modules 51 with an outer peripheral surface 60 of the dental implant model 6 located outside the implant modules 51. As shown in Fig. 10B, the outer peripheral surface 60 of the dental implant model 6 is in contact with the eight radially innermost assessment zones. The innermost assessment zones on the test modules 52 are defined as selected zones 521, while the remaining three radially outer assessment zones on each of the test modules 52 are defined as non-selected zones 522.

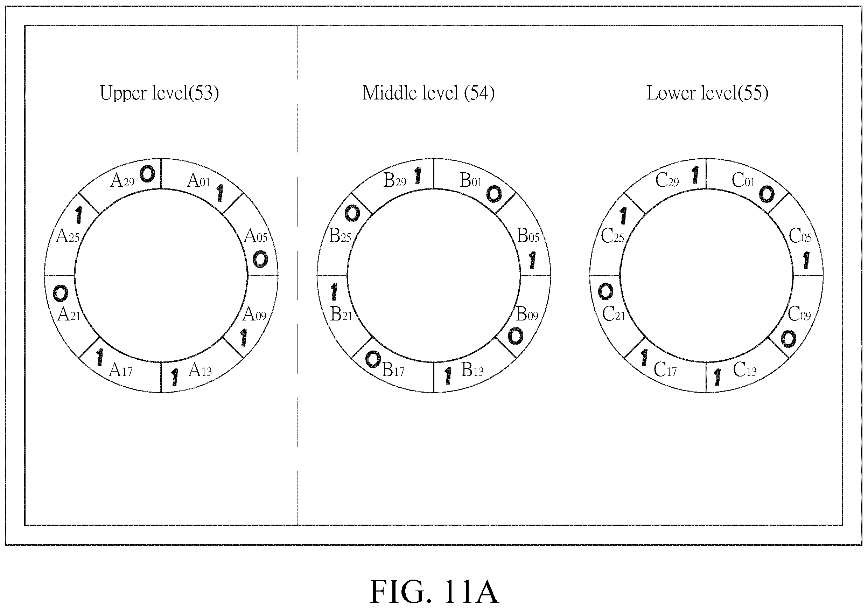

Please refer to Fig. 11A. In the result generation step S7, the selected zones 521 in the upper level 53, including the assessment zones A01, A05, A09, A13, A17, A21, A25 and A29, the selected zones 521 in the middle level 54, including the assessment zones B01, B05, B09, B13, B17, B21, B25 and B29, and the selected zones 521 in the lower level 55, including the assessment zones C01, C05, C09, C13, C17, C21, C25 and C29, are picked out and shown separately. As shown, among the selected zones 521, the assessment zones A01, A09, A13, A17, A25 in the upper level 53 respectively have a valid value [1], while other assessment zones A05, A21, A29 in the upper level 53 respectively have an invalid value [0]; the assessment zones B05, B13, B21, B29 in the middle level 54 respectively have a valid value [1], while other assessment zones B01, B09, B17, B25, B in the middle level 54 respectively have an invalid value [0]; and the assessment zones C05, C13, C17, C25, C29 in the lower level 55 respectively have a valid value [1], while other assessment zones C01, C09, C21 in the lower level 55 respectively have an invalid value [0].

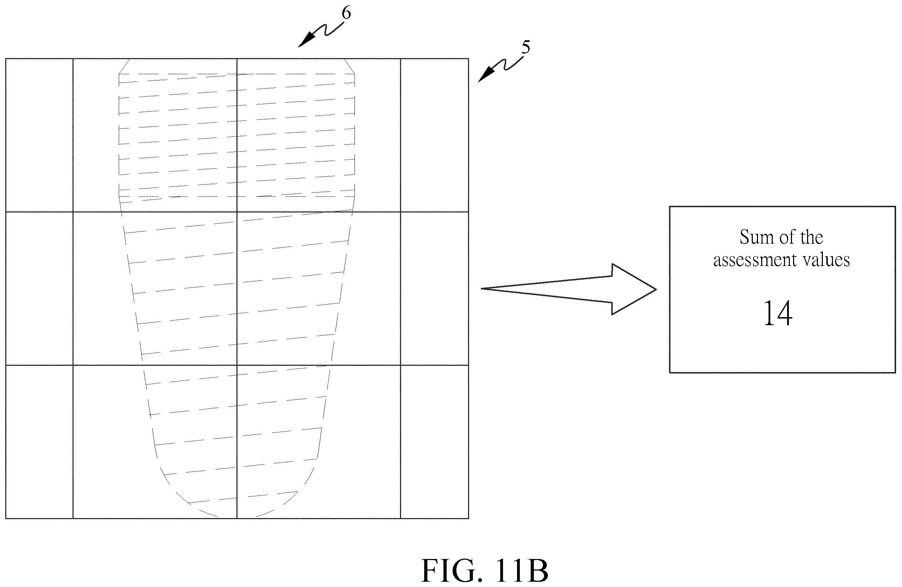

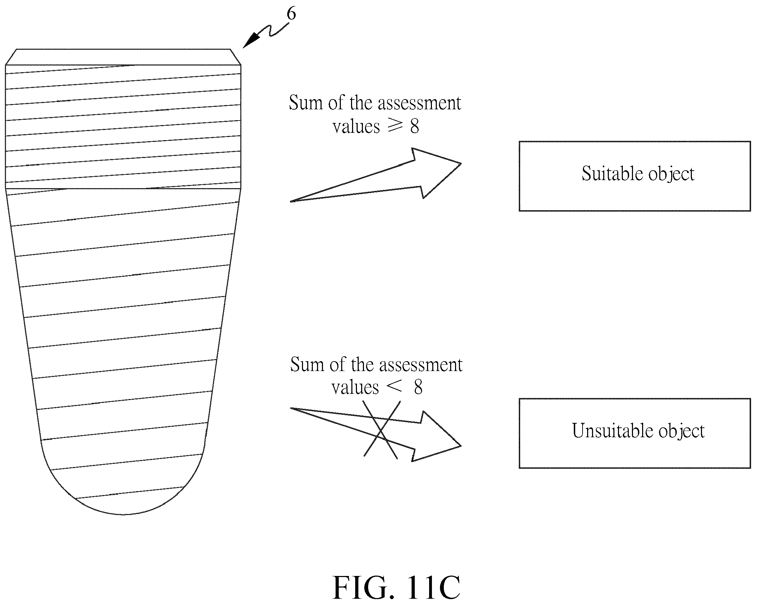

Please refer to Figs. 11B and 11C. The electronic device makes a computation on the assessment values of the selected zones 521 to derive a sum of the assessment values for determining an object type of the dental implant model 6. The dental implant model 6 and its object type will be shown on the electronic device at the same time. The dental implant model 6 may be one of the following three object types, namely, a suitable object that can be used for immediate implant placement, an unsuitable object that fails to provide stable and firm fixing ability, and an optimal object that can be used for immediate implant placement.

When the sum of the assessment values is larger than or equal to one-third of a total number of the assessment zones, the dental implant model 6 shall be determined as a suitable object. When the sum of the assessment values is smaller than one-third of the total number of the assessment zones, the dental implant model 6 is determined as an unsuitable object. When the sum of the assessment values is larger than two-third of the total number of the assessment zones, the dental implant model 6 is determined as an optimal object.

As shown in Fig. 11B, since the sum of the assessment values is 14, the dental implant model 6 is determined as a suitable object, which allows the dentist to place a dental implant corresponding to the dental implant model 6 in an extraction socket at the same time the damaged tooth is extracted. After the dental implantation, the patient needs to rest for a period of time to allow for good osseointegration between the dental implant and the patient's alveolar bone, and a subsequent procedure of mounting an artificial crown can be performed only when the good osseointegration is achieved.

In another operable embodiment, the hardness data can be an accurate hardness value and the density data can be an accurate density value. The electronic device calculates based on the hardness values and the density values to derive each of the assessment values, which would be an estimated forcing strength (not shown). The sum of the assessment values is defined as a force in newton for determining the object type of the dental implant model 6. When the force is more than 20 newtons, the dental implant model 6 is determined as a suitable object. On the other hand, when the force is less than 20 newtons, the dental implant model 6 is determined as an unsuitable object (not shown).

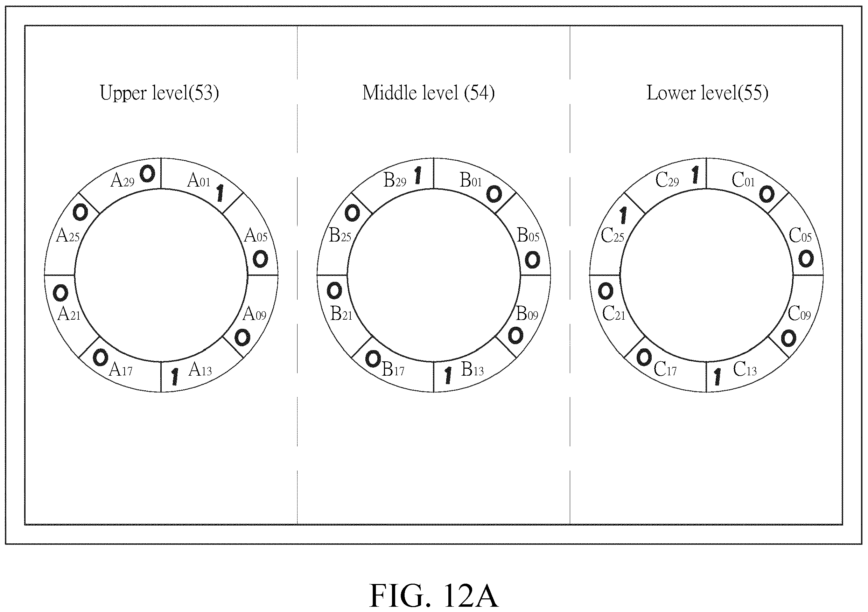

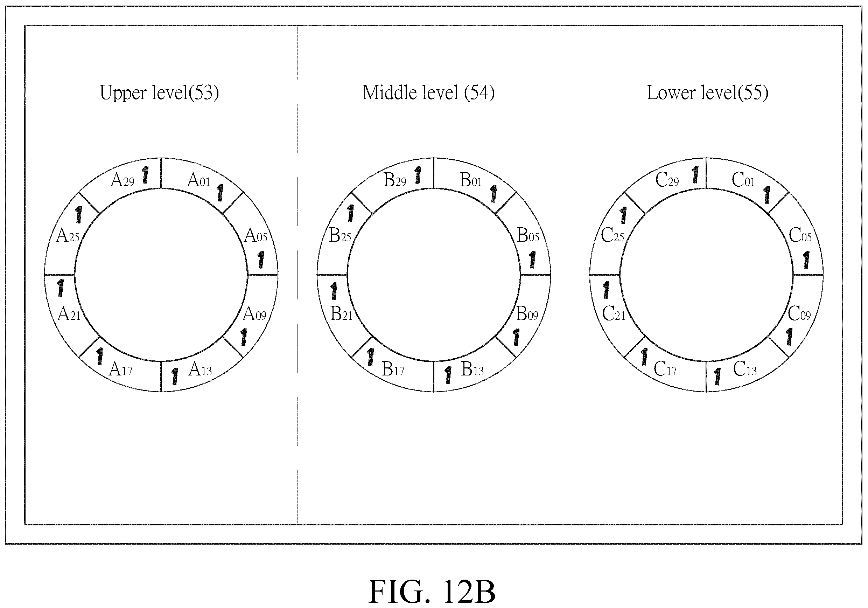

Please refer to Figs. 12A and 12B, which show a dental implant model 6 being an unsuitable object and an optimal object, respectively. As shown in Fig. 12A, only two assessment zones A01 and A13 in the upper level 53 of the dental implant model 6 have the valid value [1], only two assessment zones B13 and B29 in the middle level 54 of the dental implant model 6 have the valid value [1], and only three assessment zones C13, C25, C29 in the lower level 55 of the dental implant model 6 have the valid value [1]. In this case, the one-third of the total number of the assessment zones is 8, and the sum of the assessment values is 7, which is smaller than the one-third of the total number of the assessment zones, i.e. smaller than 8. Therefore, the dental implant model 6 in Fig. 12A is not suitable for immediate implant placement. In this case, a dental bone graft material must be filled in the extraction socket after the damaged tooth is extracted, so as to ensure good osseointegration later.

On the other hand, as shown in Fig. 12B, all the selected zones 521 in the upper, the middle and the lower level 53, 54, 55 of the dental implant model 6 have the valid value of [1], and the sum of the assessment values is 24, which is larger than the two-third of the total number of the assessment zones, i.e. larger than 16, the dental implant model 6 in Fig. 12B is therefore determined as an optimal object and a real dental implant corresponding thereto can be placed in the extraction socket immediately after the damaged tooth is extracted, and an artificial crown can be mounted to a top of the dental implant immediately after the implant placement.

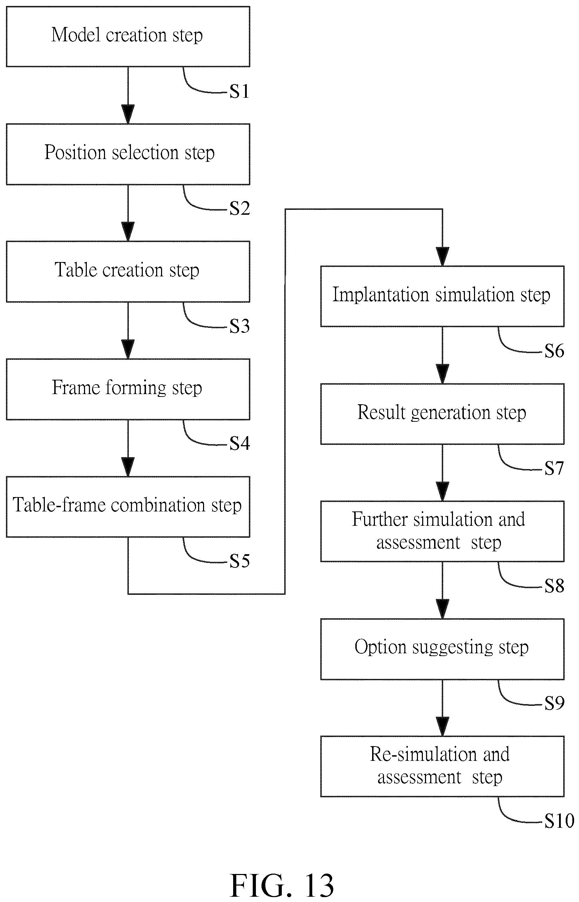

Please refer to Fig. 13. In a second preferred embodiment of the present invention, the dental implantation assessment method includes a model creation step S1, a position selection step S2, a table creation step S3, a frame forming step S4, a table-frame combination step S5, an implantation simulation step S6, a result generation step S7, a further simulation and assessment step S8, an option suggesting step S9, and a re-simulation and assessment step S10. Since the steps S1 to S7 in the second preferred embodiment are identical to those in the first preferred embodiment, they are not repeatedly described herein.

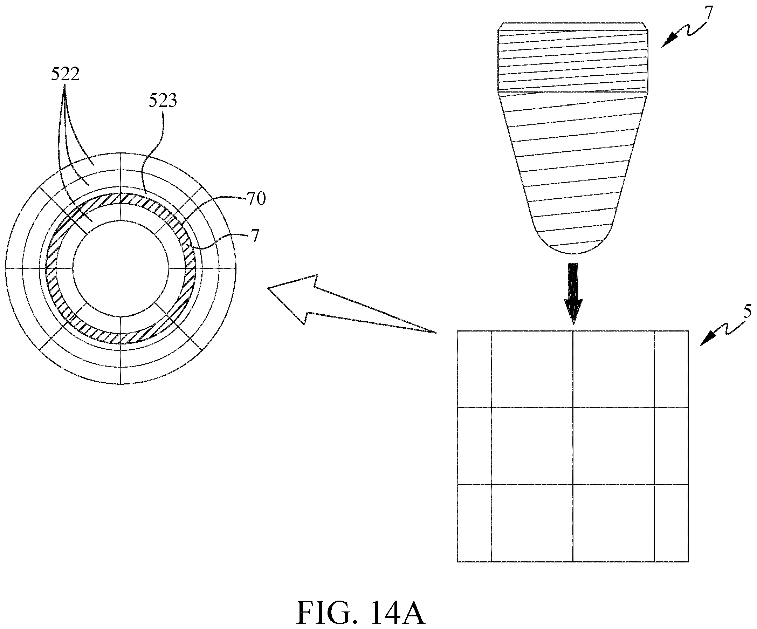

Please refer to Fig. 14A. In the further simulation and assessment step S8, a replacement dental implant model 7 is used in place of the dental implant model 6. The replacement dental implant model 7 is dimensionally larger than the dental implant model 6 and has a replacement outer peripheral surface 70, which is in contact with the test modules 52 to define a plurality of replacement selected zones 523 thereon. As shown, the replacement outer peripheral surface 70 defines total 24 replacement selected zones 523, which fall in the 24 assessment zones located at a radially second inner position of the test modules 52. The replacement selected zones 523 fall in the assessment zones A02, A06, A10, A14, A18, A22, A26, A30 in the upper level 53, the assessment zones B02, B06, B10, B14, B18, B22, B26, B30 in the middle level 54, and the assessment zones C02, C06, C10, C14, C18, C22, C26, C30 in the lower level 55.

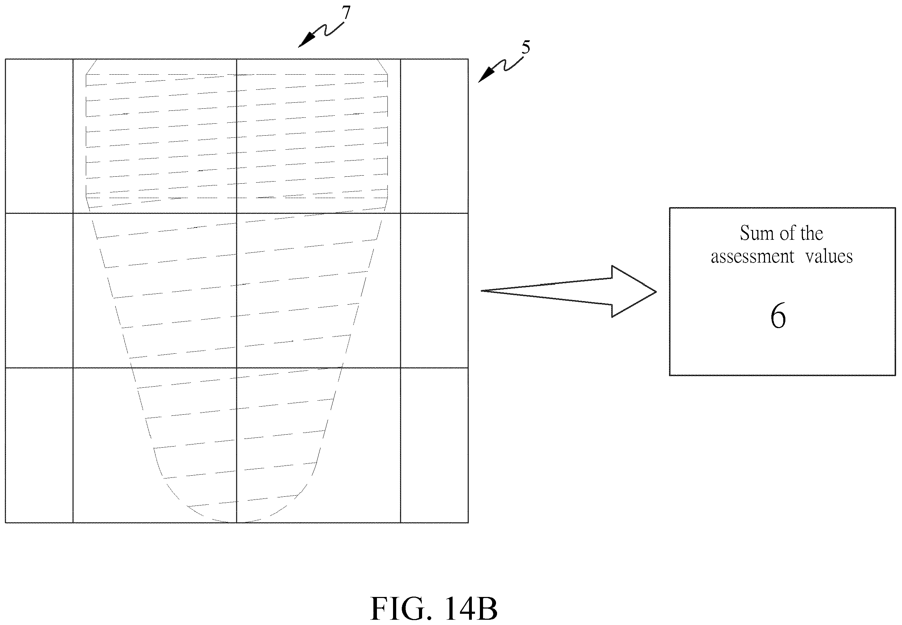

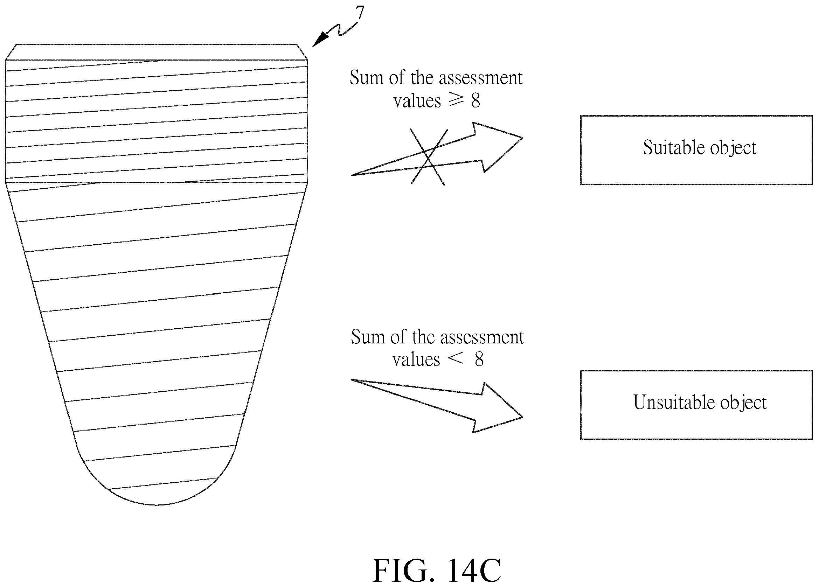

Please refer to Figs. 14B and 14C. As shown, the one-third of the total number of the assessment zones in the radially second inner position is 8, and the finally calculated sum of the assessment values of the replacement selected zones 523 is 6, which is smaller than the one-third of the total number of the assessment zones, i.e. smaller than 8. Therefore, the replacement dental implant model 7 is determined as an unsuitable object. The replacement dental implant model 7 and its object type as an unsuitable object will be shown on the electronic device at the same time.

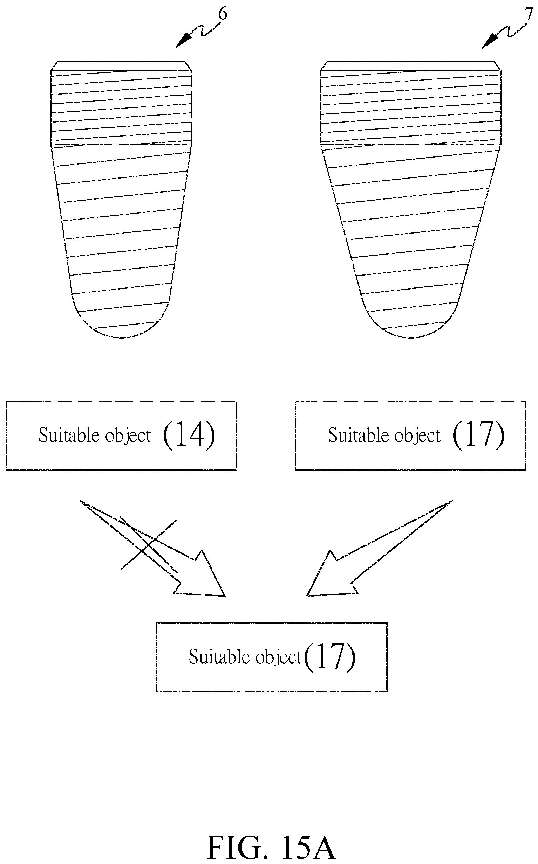

In the option suggesting step S9, the object types of the previous dental implant model 6 and the replacement dental implant model 7 are compared to generate a suggested option. Please refer to an example in Fig. 15A. The sum of the assessment values of the dental implant model 6 is 14, while the sum of the assessment values of the replacement dental implant model 7 is 17. When both of the dental implant model 6 and the replacement dental implant model 7 are suitable objects, the one having a higher sum of the assessment values shall be selected as the suggested option for use in the dental implant surgery. In the illustrated case, the replacement dental implant model 7 is selected as the suggested option for the dental implant surgery.

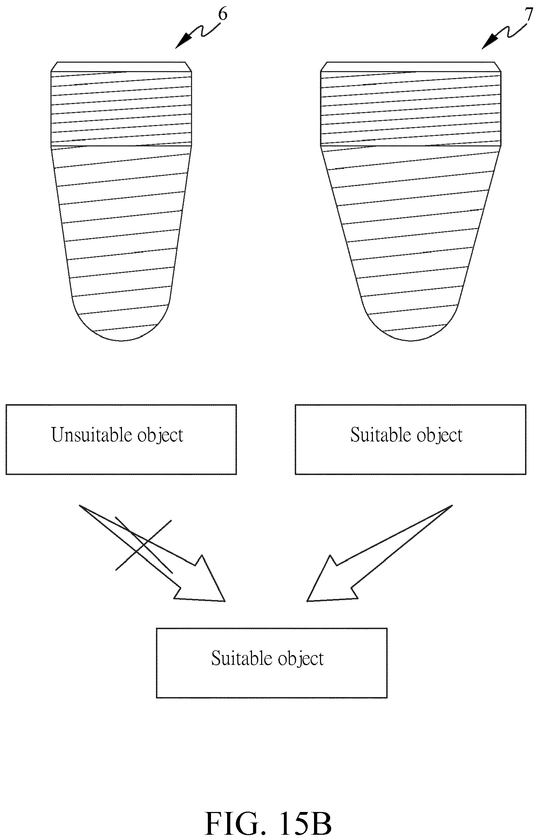

Please refer to Fig. 15B. When one of the dental implant model 6 and the replacement dental implant model 7 is determined as a suitable object while the other one is an unsuitable object, the comparison of the sums of the assessment values can be omitted and the one model being the suitable object is directly selected as the suggested option for use in the dental implant surgery.

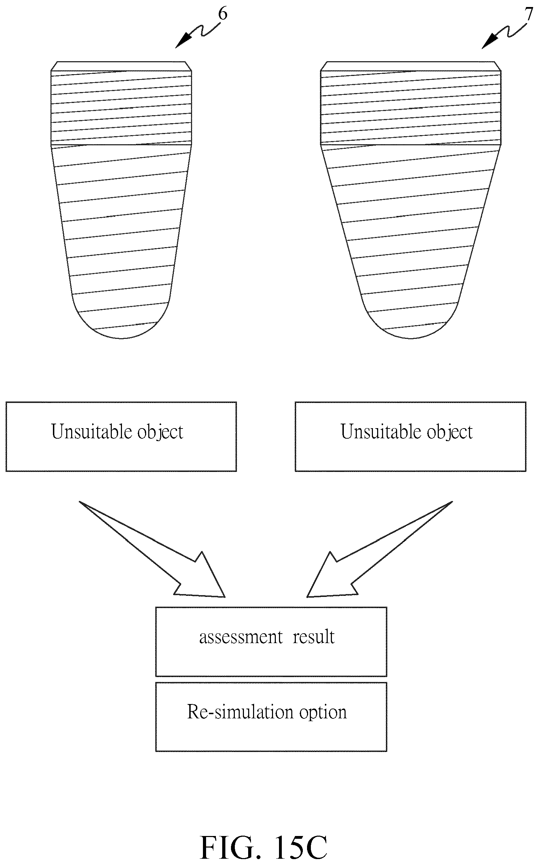

Please refer to Fig. 15C. When the dental implant model 6 and the replacement dental implant model 7 all are unsuitable objects, the suggested option includes an assessment result that no dental implant surgery can be performed and a re-simulation option that a re-simulation and assessment step S 10 is to be performed.

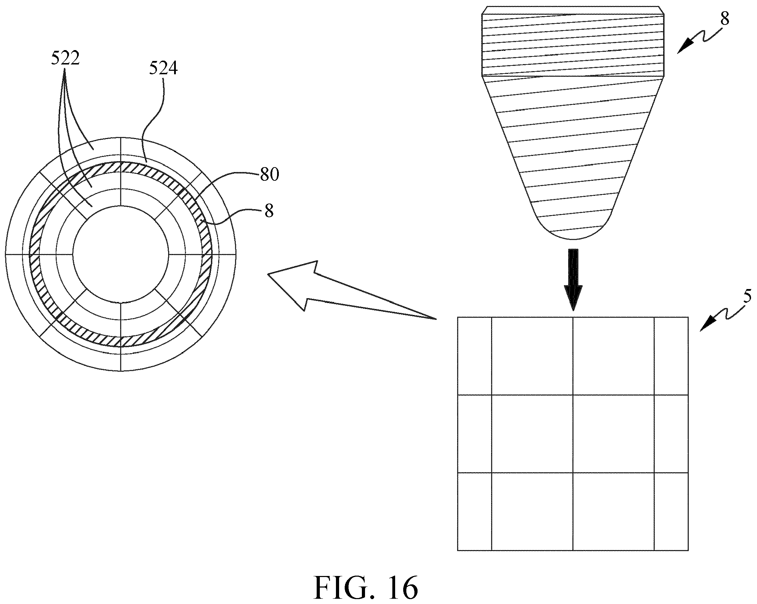

Please refer to Fig. 16. In the re-simulation and assessment step S10, a further replacement dental implant model 8 is used in place of the replacement dental implant model 7. The further replacement dental implant model 8 is dimensionally larger than the replacement dental implant model 7 and has a further replacement outer peripheral surface 80, which is in contact with the test modules 52 to define a plurality of further replacement selected zones 524. As shown, the further replacement outer peripheral surface 80 defines total 24 further replacement selected zones 524, which fall in the 24 assessment zones located at a radially third inner position of the test modules 52. The further replacement selected zones 524 fall in the assessment zones A03, A07, A11, A15, A19, A23, A27, A31 in the upper level 53, the assessment zones B03, B07, B11, B15, B19, B23, B27, B31 in the middle level 54, and the assessment zones C03, C07, C11, C15, C19, C23, C27, C31 in the lower level 55. Lastly, the sum of the assessment values of the further replacement selected zones 524 is derived and used to determine the object type of the further replacement dental implant model 8.

## Claims

1. A dental implantation assessment method for implant dentistry, comprising:
a model creation step, in which a computed tomography (CT) device is used to scan a patient's oral areas for getting a plurality of images, and a teeth model having a three-dimensional (3D) appearance is created on an electronic device based on the images;
a position selection step, in which an implant placement position is determined on the teeth model by the electronic device and a reference line passing a center of dental occlusion is set up at the implant placement position;
a table creation step, in which an alveolar bone area on the teeth model located around the reference line is determined on the electronic device and a health status table is generated by electronic device based on the alveolar bone area; and the health status table having a plurality of hardness data and a plurality of density data for listing in different regions of the alveolar bone area;
a frame forming step, in which a frame model is formed on the electronic device at the implant placement position around the reference line, such that the alveolar bone area is encircled by the frame model; and the frame model internally defining a plurality of levels, each of the levels including an implant module in the center and a plurality of test modules located immediately around the implant module;
a table-frame combination step, in which every test module is divided into a plurality of assessment zones on the electronic device, and the assessment zones being combined with the hardness data and the density data of the health status table; an assessment value of the assessment zones are calculated by the electronic device according to the health status table; and each of the assessment values in the corresponding assessment zone being decided to one of a valid value representing a healthy state and an invalid value representing an unhealthy state;
an implantation simulation step, in which a dental implant model is selected by the electronic device for placement into the frame model, such that the implant module of the levels overlap the dental implant model, and one of the assessment zones of every test module adjacent to an outer peripheral surface of the dental implant model is defined as a selected zone; and
a result generation step, in which the electronic device makes a computation on assessment every selected zone to derive a sum value of the assessment values for determining an object type of the dental implant model; the dental implant model being determined as a suitable object allowed for use in immediate implant placement when the sum value is larger than a specified proportion of a total number of the assessment zones; and the dental implant model being determined as an unsuitable object failing to provide stable and firm fixing ability when the sum value is smaller than the specified proportion of the total number of the assessment zones.

2. The dental implantation assessment method for implant dentistry as claimed in claim 1, further comprising a further simulation and assessment step, in which a replacement dental implant model is used on the electronic device in place of the dental implant model; the replacement dental implant model being different from the dental implant model in size and having a replacement outer peripheral surface; the assessment zones on every test module adjacent to the replacement outer peripheral surface being defined as replacement selected zones, according to which an object type of the replacement dental implant model is determined.

3. The dental implantation assessment method for implant dentistry as claimed in claim 2, further comprising an option suggesting step, in which the electronic device makes a comparison between the object type of the dental implant model and the replacement dental implant model to generate a suggested option.

4. The dental implantation assessment method for implant dentistry as claimed in claim 3, wherein, in the case the dental implant model and the replacement dental implant model all are determined as suitable objects, one of the suitable objects having a higher sum value of the assessment values is selected as the suggested option for use in the dental implant surgery; and in the case one of the dental implant model and the replacement dental implant model is determined as a suitable object while the other one is an unsuitable object, the one being the suitable object is selected as the suggested option for use in the dental implant surgery.

5. The dental implantation assessment method for implant dentistry as claimed in claim 3, wherein, in the case both of the dental implant model and the replacement dental implant model are determined as unsuitable objects, the suggested option includes an assessment result that no dental implant surgery can be performed and a re-simulation option that a re-simulation and assessment step is to be performed.

6. The dental implantation assessment method for implant dentistry as claimed in claim 5, wherein, in the re-simulation and assessment step, a further replacement dental implant model is used on the electronic device in place of the replacement dental implant model; the further replacement dental implant model being different from the replacement dental implant model in size and having a further replacement outer peripheral surface; the assessment zones on every test module adjacent to the further replacement outer peripheral surface being defined as further replacement selected zones, so that an object type of the further replacement dental implant model is determined based on the further replacement selected zones.

7. The dental implantation assessment method for implant dentistry as claimed in claim 1, wherein the frame model has a cross-sectional diameter that is limited by one of the following two measures: a distance between two teeth located immediately at two lateral sides of the implant placement position, and a width of gum of the teeth model.

8. The dental implantation assessment method for implant dentistry as claimed in claim 7, wherein the dental implant model has an implant diameter and an implant length; the implant diameter is limited by an area size of the frame model, and the implant length is limited by the number of levels of the frame model.

9. The dental implantation assessment method for implant dentistry as claimed in claim 1, wherein, in the frame forming step, a height location of the frame model is determined according to a top 3D configuration of the alveolar bone area; and the top 3D configuration defines a slanted reference surface, the slanted reference surface angularly intersects the reference line and the frame model has a top surface that passes an intersection of the slanted reference line and the reference line.

10. The dental implantation assessment method for implant dentistry as claimed in claim 1, wherein, in the frame forming step, a tilting direction of the frame model is determined according to the health status of the alveolar bone area; and the frame model having a centerline that is inclined relative to the reference line, such that a plurality of contact volumes of the frame model with the alveolar bone area are changed to different positions.

11. A dental implantation assessment method for implant dentistry, comprising:
a model creation step, in which a computed tomography (CT) device is used to scan a patient's oral areas for getting a plurality of images and a teeth model having a three-dimensional (3D) appearance is created on an electronic device based on the images;
a position selection step, in which an implant placement position on the teeth model is determined on the electronic device and a reference line passing a center of dental occlusion is set up at the implant placement position;
a table creation step, in which an alveolar bone area on the teeth model located around the reference line is determined on the electronic device and a health status table is generated by electronic device based on the alveolar bone area; and the health status table having a plurality of hardness data and a plurality of density data for listing in different regions of the alveolar bone area;
a frame forming step, in which a frame model is formed on the electronic device at the implant placement position around the reference line, such that the alveolar bone area is encircled by the frame model; and the frame model internally defining a plurality of levels, each of the levels including an implant module in the center and a plurality of test modules located immediately around the implant module;
a table-frame combination step, in which every test module is divided into a plurality of assessment zones on the electronic device; and the assessment zones being combined with the hardness data and the density data of health status table; an assessment value of the assessment zones are calculated by the electronic device according to the health status table; and each of the assessment values being a fixing force acting between a dental implant model and the alveolar bone area;
an implantation simulation step, in which the dental implant model is selected by the electronic device for placement into the frame model, such that the implant module of the levels overlap the dental implant model and one of the assessment zones of every test module adjacent to an outer peripheral surface of the dental implant model is defined as a selected zone; and
a result generation step, in which the electronic device makes a computation on the every selected zone to derive a sum value of the assessment values for determining an object type of the dental implant model; the dental implant model being determined as a suitable object allowed for use in immediate implant placement when the sum value is larger than a specified force; and the dental implant model being determined as an unsuitable object failing to provide stable and firm fixing ability when the sum value is smaller than the specified force.
